# EUROPEAN PATENT APPLICATION

(11) **EP 1 342 732 A1**
(43) Date of publication of application: **10.09.2003**
(21) Application number: 03251270.9
(22) Date of filing: 04.03.2003
(51) Int. Cl.: C08B 11/20, C08L 1/28, A61K 47/38, A61K 9/06

(54) **Pharmaceutical carrier comprising low-substituted cellulose ether, for external application**

(30) Priority: 04.03.2002 JP 2002056995
(71) Applicant: Shin-Etsu Chemical Co., Ltd., Chiyoda-ku Tokyo 100 (JP)
(72) Inventor: Obara, Sakae, c/o Shin-Etsu Chemical Co. Ltd., Naka Kubiki-gun, Niigata-ken (JP)
(74) Representative: Goddard, David John

(57) **Abstract**

A pharmaceutical carrier for external application comprises a low-substituted alkyl and/or hydroxyalkyl cellulose ether, where the degree of substitution is 0.05 to 1.0 molar. The carrier is stable even in the presence of alcohol, interacts less with an active ingredient that an ionic carrier, and feels pleasant. It may be made by a method including the step of swelling and dispersing it in water for shear trituration, or by dissolving it in an alkaline solution for trituration during or after neutralisation by acid.

## Description

### BACKGROUND OF THE INVENTION

### 1. Field of the invention

The present invention relates to carriers used for pharmaceutical dosage forms for external application.

### 2. Description of the related art

Conventionally, fats and oils, emulsifiable concentrates, water-soluble polymers and the like have been employed as carriers used for pharmaceutical dosage forms for external application. Among them, thickening agents such as carboxyvinyl polymers have been used as water-soluble polymers, providing a refreshing feeling because of transparency thereof, good feeling when used because of good spreading without tackiness and an advantage that their active ingredient has good absorbability. Also, Japanese Patent No. 2610052 (USP No. 5086077) discloses a carrier for external application in which cellulose ether modified with hydrophobic groups is used.

### SUMMARY OF THE INVENTION

Qualities required for pharmaceutical dosage forms for external application include good absorbability of the active ingredient and a good feeling when used as previously described. Carboxyvinyl polymers provide a good feeling when used. However, polymers are problematic in that they produce complexes with certain drugs, particularly with basic drugs because they are ionic, thereby inhibiting the absorption of the drugs.

The pharmaceutical carrier for external application using the hydrophobic group-modified cellulose ether described in Japanese Patent No. 2610052 has an advantage that it is not easy to undergo an interaction with other drugs because the polymer of the major ingredient is non-ionic. On the other hand, this polymer expresses thixotropic viscosity by a hydrophobic interaction of long-chain alkyl groups and thus has characteristics providing a good feeling when used along with good spreading. However, the hydrophobic interaction is offset by the addition of an organic solvent such as alcohol. Particularly, there has been a drawback that in the case of intending to enhance the absorbability of the active ingredient, although an organic solvent such as alcohol is necessary to be added in order to increase solubility of the drug in the system, the thixotropic viscosity is offset thereby.

The present invention has been carried out in light of the above issues, and is intended to provide a pharmaceutical carrier for external application which is stable and provides a good feeling in use even when alcohol or the like is added and of which the major ingredient is a non-ionic polymer which interacts less with an active ingredient.

As a result of an intensive study to achieve the above objectives, the present inventors have found that the above objectives are achieved by formulating low-substituted cellulose ether which is a non-ionic polymer and interacts less with an active ingredient, and have completed the present invention.

The present invention provides a pharmaceutical carrier for external application comprising a low-substituted cellulose ether with the degree of molar substitution with alkyl and/or hydroxyalkyl groups being 0.05 to 1.0. The low-substituted cellulose ether may be preferably in a swelled state where particles thereof are evenly dispersed. Also, the present invention provides a method for producing the pharmaceutical carrier for external application comprising a step of swelling and dispersing a low-substituted cellulose ether having a degree of molar substitution of 0.05 to 1.0 into water by shear-trituration, as well as a method for producing a pharmaceutical carrier for external application comprising a step of dissolving a low-substituted cellulose ether having a degree of molar substitution of 0.05 to 1.0 in an alkali aqueous solution and a step of shear-trituration during or after neutralizing by the addition of an acid thereto.

The degree of molar substitution with an alkyl and/or hydroxyalkyl group can be measured with reference to Pharmacopoeia of Japan.

According to the present invention, it is possible to obtain a pharmaceutical carrier for external application which is stable, feels good in use even when alcohol or the like is added, and interacts less with the active ingredient.

### DETAILED DESCRIPTION OF THE PREFERRED EMBODIMENTS

The present invention is explained in greater detail below.

The low-substituted cellulose ether used in the present invention is one wherein the substituent is an alkyl and/or hydroxyalkyl group and has a property of not dissolving but of swelling in water.

Generally, celluloses are insoluble in water, but when hydrogen atoms of hydroxyl groups in glucose rings composing the cellulose are substituted with alkyl and hydroxyalkyl groups, they tend to have solubility in water depending on the degree of substitution. However, those having a low degree of substitution are not observed having solubility in water. In many cases, the low-substituted cellulose ether powder becomes to be in the partially swelling state when dispersed in water. When its degree of substitution is high, it becomes water-soluble and conversely loses a dissolving property in alkali. When the high-substituted cellulose ether is used, the carrier of the present invention cannot be obtained.

The degree of molar substitution with an alkyl and/or hydroxyalkyl group of the low-substituted cellulose ether used in the present invention is 0.05 to 1.0. Examples of various low-substituted cellulose ethers having a desired content of substituents (degrees of molar substitution are shown in parentheses) include low-substituted methyl cellulose having 3 to 15% by weight of methoxyl groups (0.16 to 0.85), low-substituted hydroxyethyl cellulose having 3 to 15% by weight of hydroxyethoxyl groups (0.08 to 0.45), low-substituted hydroxypropyl cellulose having 4 to 20% by weight of hydroxypropoxyl groups (0.09 to 0.51), low-substituted hydroxypropylmethyl cellulose having 3 to 12% by weight of methoxyl groups and 4 to 20% by weight of hydroxypropoxyl groups (0.25 to 1.0, together with both substituents), and the like.

In this manner, the low-substituted cellulose ether has a property that makes it insoluble in water but allows it to dissolve in alkali and swells by absorbing water.

A more typical example includes low-substituted hydroxypropyl cellulose. Currently, this material is commercially available under the trade name of L-HPC by Shin-Etsu Chemical Co., Ltd. It is described in Pharmacopoeia of Japan, and widely used as a disintegrant formulated in tablets in the pharmaceutical material field.

These low-substituted cellulose ethers can be produced by methods known in the art as disclosed in Japanese Patent Publication (JP-B) No. 57-53100 (USP No. 4091205).

Specifically, first preparation of alkali cellulose may be required. This may be prepared by immersing sheets of pulp which are starting materials in an aqueous alkali solution, e.g., caustic soda, or mixing pulverized pulp with the alkali solution, or dispersing pulp powder in an organic solvent followed by adding the alkali.

Next, the alkali cellulose may be placed in a reactor, then an etherifying agent such as propylene oxide or ethylene oxide may be added, and subsequently, the mixture may be heated and reacted to yield cellulose ether. The crude cellulose ether after the reaction may be transferred to another tank, and the alkali may be neutralized with an acid to yield a solid, which may be washed, dried and pulverized to afford a final product as powder. Alternatively, there may be also a case where a method is employed in which the crude cellulose ether immediately after the reaction may be dissolved completely or partially in water, which may be neutralized, and subsequently a polymer to be precipitated may be exclusively batched off, washed, dried and pulverized.

In the method for producing the pharmaceutical carrier for external application of the present invention, it may be sufficient to disperse powder (preferably the average particle diameter is 0.1 to 100 µm) of the low-substituted cellulose ether as it is in other water or in a mixture system of water and alcohol, and mix evenly. However preferably, the low-substituted cellulose ether may be added followed by shear-trituration using a dispersion apparatus such as an emulsifying dispersion machine. This allows the cellulose ether particles to highly swell with water and to increase viscosity.

Also, a more preferable method is a method where the low-substituted cellulose ether may be once dissolved in the alkali, and shear-triturated using the emulsifying dispersion machine while or after the solution is neutralized by the addition of the acid thereto. The precipitated cellulose ether particles in this manner may possess a sharp distribution in particle sizes with a high degree of swelling, possess thixotropic viscosity, and be smooth and feel excellent when used.

Alkalis used for dissolution include caustic potash, caustic soda and the like, and its concentration may be appropriately determined since it varies depending on the types and degrees of substitution of the substituents of the cellulose ether used. In a typical example, a low-substituted hydroxypropyl cellulose with a 0.2 degree of molar substitution is dissolved in 10% by weight of caustic soda. Depending on the difference in the substituent distribution, there are some cases where it becoms a completely clear solution and cases where it is not completely clear solution. In the latter case, when the viscosity is obviously increased, it is considered to be dissolved.

For neutralization of the alkali solution, it may be sufficient to use an equivalent of hydrochloric acid, sulfuric acid, acetate acid or the like. The alkali solution is subjected to a shear-trituration machine, and shear-triturated while carrying forward the neutralization by gradually adding the acid. Or the alkali solution is neutralized by an equivalent acid in advance and shear-triturated in the presence of the precipitated polymer.

The shear-trituration is a method where the alkali solution of the cellulose ether at an appropriate concentration or the neutralized solution thereof may be preferably triturated using the emulsifying dispersion machine. Emulsifying dispersion machines are not particularly limited, and include a vibration ball mill, colloid mill, homomixer, homogenizers such as a propeller homogenizer, high pressure homogenizer and an ultrasonic homogenizer. Among them, preferred is the homogenizer. The high pressure homogenizer where a treatment liquid is emitted via joint gaps of valves (e.g., "Homogenizer" supplied by Sanwa Machine Co., Inc., "Ultimizer System" supplied by Sugino Machine Co., Ltd., "Microfluidizer" supplied by Mizuho Industrial Co., Ltd., high pressure homogenizer supplied by Gorlin Co., Ltd.), and the ultrasonic homogenizer utilizing ultrasonic oscillations (e.g., "Ultrasonic Homogenizer" supplied by Nippon Seiki Co. Ltd.) are preferred to prepare a homogenous water dispersing system.

At this time, in the case of the alkali solution, the trituration may be carried forward by gradually adding acid such as hydrochloric acid, sulfuric acid or acetic acid. The shear-trituration condition is not particularly limited, but the propeller homogenizer may be used preferably at pressure from 3,000 to 15,000 rpm and more preferably from 10,000 to 15,000 rpm. The high pressure homogenizer may be used preferably at pressure from 100 to 2,000 kg/cm² and more preferably 200 to 2,000 kg/cm².

Also, a similar effect may be obtained by re-dispersing the powder yielded by spray-drying of the low-substituted cellulose ether dispersion produced by the above methods. The spray-drying may be carried out, for example, by drying at 20 to 150°C using a spray-drying apparatus.

In addition, without dissolving the low-substituted cellulose ether in the alkali as described above, it is possible to use a method where the low-substituted cellulose ether (preferably the average particle diameter is 0.1 to 100 µm) is swelled and dispersed in water followed by shear-trituration, preferably shear-trituration using an emulsifying dispersion machine.

The concentration of cellulose ether in the solution when shear-triturated may be preferably 0.01 to 20% by weight and especially 0.1 to 10% by weight. If the concentration is higher than this, the viscosity may be excessively increased and the trituration may become difficult.

Since salts produced by the neutralization are present in the prepared dispersion, these may be eliminated by techniques such as centrifugation or dialysis if desired. The dispersions of the present invention also include those which are a gel.

These dispersions may become carriers by formulating other ingredients.

The concentration of cellulose ether in the carrier is preferably 0.1 to 50% by weight, and 0.5 to 20% by weight is especially preferred. When the concentration is less than 0.1% by weight, sufficient viscosity may not be obtained. When it is more than 50% by weight, the production of the uniform product may become difficult because of the excessively high viscosity thereof.

The pharmaceutical carrier for external application of the present invention comprises the low-substituted cellulose ether, while other ingredients thereof are not particularly limited. Specifically, water and the water/alcohol mixture may be used as dispersion media.

Also, a moisturizer such as polyhydric alcohol, a sorbefacient, a preservative, perfume, fat and oil or the like may be comprised if desired.

Depending on the purposes, an active ingredient such as an antibiotic, a chemotherapeutic agent, a vitamin preparation, a topical anesthetic, an anti-histamine agent, an antiphlogistic analgesic, an astringent, sulfa, an antifungal, a blood flow enhancing agent or an adrenal cortex hormone agent can be added to the carrier according to the present invention.

The present invention is explained below by showing examples and comparative examples, but the invention is not limited to the following examples.

### Production Example 1 (Production 1 of polymer stock solution)

A suspension comprising 3 weight parts of the low-substituted hydroxypropyl cellulose (LH-21 supplied by Shin-Eetsu Chemical Co., Ltd.) where the degree of molar substitution of a hydroxypropoxyl group is 0.23 and 97 weight parts of water was shear-triturated by being passed three times at a pressure of 1,750 kg/cm² through a high pressure homogenizer (Microfluidizer supplied by Fluidex of the USA). The resultant dispersion was centrifuged to a concentration of 7% by weight of a solid to yield an aqueous gel. Production Example 2 (Production 2 of polymer stock solution)

The low-substituted hydroxypropyl cellulose where the degree of molar substitution of hydroxypropoxyl groups is 0.23 was dissolved in 10% by weight of caustic soda. The polymer concentration was 6% by weight at this time. While this solution was shear-triturated at 5,000 rpm using a homogenizer (AM-10 type supplied by Nippon Seiki Co., Ltd.), while hydrochloric acid was dripped until being neutralized. After the neutralization was over, the shear-triturating was further continued at 10,000 rpm for 10 min. Salts were removed from the resultant gel by centrifugation, and the aqueous gel was yielded by preparing a polymer concentration that was 7% by weight.

### Example 1 (Production of pharmaceutical carrier for external application)

A pharmaceutical carrier for external application was prepared by the following formula, and consequently, gave good feeling when used.

| 7% by weight of the aqueous gel obtained in Production | | |
|---|---|---|
| Example 1 | | 70 parts by weight |
| | Ethyl alcohol | 15 parts by weight |
| | Propylene glycol | 15 parts by weight |

### Example 2 (Preparation of pharmaceutical carriers for external application, effects of alcohol)

Carriers were prepared by the following formulae a and b. Viscosity scarcely differed between Formula a and Formula b, and these carriers were excellent in good spreading.

| <Formula a> | | |
|---|---|---|
| | 7% by weight of the aqueous gel obtained in Production | |

| Example 2 | | 70 parts by weight |
|---|---|---|
| | Water | 30 parts by weight |
| Viscosity: 9,200 mpa·s (at 20°C, Brookfield viscometer No.4 rotor, 30 rpm) | | |

| <Formula b> | | |
|---|---|---|
| | 7% by weight of the aqueous gel | obtained in Production |

| Example 2 | | 70 parts by weight |
|---|---|---|
| | Ethyl alcohol | 30 parts by weight |
| Viscosity: 9,100 mPa·s (at 20°C, Brookfield viscometer No.4 rotor, 30 rpm) | | |

### Comparative Example 1 (Production of pharmaceutical carriers for external application, effects of alcohol)

Carriers were prepared by the following Formula c and Formula d. Formula c produced a carrier that gave good feeling when used, while viscosity based on Formula d was lower and shape retention was inferior. The spreading based on Formula d was poor even when the amount of the polymer was increased to elevate viscosity. The carrier for Formula d gave inferior feeling when used.

| <Formula c> | | |
|---|---|---|
| | Hydrophobically-modified Hydroxypropyl Methylcellulose | |
| | | 2 weight parts |
| | (Degree of molar substitution of 0.006) | a stearyl group is |
| | Water | 98 weight parts |
| Viscosity: 15,000 mPa·s (at 20°C, No.4 rotor, 30 rpm) Brookfield viscometer | | |

| <Formula d> | | |
|---|---|---|
| | Hydrophobically-modified Hydroxypropyl Methylcellulose | |
| | | 2 parts by weight |
| | (Degree of molar substitution of a stearyl group is 0.006) | |
| | Water | 98 parts by weight |
| | Ethyl alcohol | 30 parts by weight |
| Viscosity: 420 mPa·s (at 20°C, Brookfield viscometer No.4 rotor, 30 rpm) | | |

### Example 3 (Production of pharmaceutical dosage form for external application)

A pharmaceutical dosage form for external application was produced by the following formula, and consequently, gave good feeling when used.

| | | |
|---|---|---|
| 7% by weight of the aqueous gel obtained in Production | | |

| Example 2 | | 60 parts by weight |
|---|---|---|
| | Isopropyl alcohol | 39 parts by weight |
| | Indomethacin | 1 parts by weight |

### Example 4 (Production of pharmaceutical dosage form for external application)

A pharmaceutical dosage form for external application was produced by the following formula, and consequently, gave good feeling when used.

| 7% by weight of the aqueous gel obtained in Production | | |
|---|---|---|
| Example 2 | | 60 parts by weight |
| | Isopropyl alcohol | 20 parts by weight |
| | Water | 20 parts by weight |
| | Tetracycline hydrochloride | 0.1 parts by weight |

### Comparative Example 2 (Production of pharmaceutical dosage form for external application)

A pharmaceutical dosage form for external application was produced by the following formula. White precipitates were generated after the production.

| | |
|---|---|
| Carboxyvinyl polymer | 2 parts by weight |
| Water | 76 parts by weight |
| Isopropyl alcohol | 20 parts by weight |
| Tripropanol amine | 1.4 parts by weight |
| Tetracycline hydrochloride | 0.1 parts by weight |

## Claims

1. A pharmaceutical carrier for external application comprising a low-substituted cellulose ether with a degree of molar substitution with an alkyl and/or hydroxyalkyl group being 0.05 to 1.0.

2. The pharmaceutical carrier for external application according to Claim 1, wherein particles of said low-substituted cellulose ether are in a swelled state and evenly dispersed.

3. The pharmaceutical carrier for external application according to Claim 1 or 2 produced by a method comprising a step of swelling and dispersing a low-substituted cellulose ether having a degree of molar substitution of 0.05 to 1.0 in water by shear-trituration.

4. The pharmaceutical carrier for external application according to Claim 1 or 2 produced by a method comprising a step of dissolving a low-substituted cellulose ether having a degree of molar substitution of 0.05 to 1.0 in an alkali aqueous solution and a step of shear-triturating during or after neutralization by the addition of an acid thereto.

5. The pharmaceutical carrier for external application according to any one of Claims 1 to 4, wherein said low-substituted cellulose ether is a low-substituted hydroxypropyl cellulose having a degree of molar substitution of 0.09 to 0.51.

6. A pharmaceutical dosage form for external application comprising the pharmaceutical carrier according to any one of claims 1 to 5.

7. A method for producing a pharmaceutical carrier for external application comprising the step of shear-triturating a low-substituted cellulose ether having a degree of molar substitution of 0.05 to 1.0 in an aqueous medium.

8. A method for producing a pharmaceutical carrier for external application comprising the step of swelling and dispersing a low-substituted cellulose ether having a degree of molar substitution of 0.05 to 1.0 in water by shear-trituration.

9. A method for producing a pharmaceutical carrier for external application comprising the steps of dissolving a low-substituted cellulose ether having a degree of molar substitution of 0.05 to 1.0 in an alkali aqueous solution and shear-triturating during or after the addition of an acid thereto.

10. A method according to any one of claims 6 to 9 wherein the cellulose is substituted with alkyl and/or hydroxyalkyl groups.
